(19) Europäisches Patentamt
European Patent Office
Office européen des brevets

(11) EP 1 587 368 B1

(12) EUROPÄISCHE PATENTSCHRIFT

(45) Veröffentlichungstag und Bekanntmachung des Hinweises auf die Patenterteilung:
**27.05.2009 Patentblatt 2009/22**

(21) Anmeldenummer: **04700127.6**

(22) Anmeldetag: **05.01.2004**

(51) Int Cl.:
***A01N 53/00*** *(2006.01)* ***A01N 55/00*** *(2006.01)*
***A01N 37/34*** *(2006.01)* ***A01N 37/38*** *(2006.01)*
***A01N 31/14*** *(2006.01)*

(86) Internationale Anmeldenummer:
**PCT/EP2004/000017**

(87) Internationale Veröffentlichungsnummer:
**WO 2004/064522 (05.08.2004 Gazette 2004/32)**

(54) **REPELLENTMITTEL**

REPELLENT

REPULSIF

(84) Benannte Vertragsstaaten:
**AT BE BG CH CY CZ DE DK EE ES FI FR GB GR HU IE IT LI LU MC NL PT RO SE SI SK TR**

(30) Priorität: **17.01.2003 DE 10301906**

(43) Veröffentlichungstag der Anmeldung:
**26.10.2005 Patentblatt 2005/43**

(73) Patentinhaber: **Bayer Animal Health GmbH**
**51368 Leverkusen (DE)**

(72) Erfinder:
- **MENCKE, Norbert**
**51381 Leverkusen (DE)**
- **STANNECK, Dorothee**
**42655 Solingen (DE)**
- **TURBERG, Andreas**
**42781 Haan (DE)**
- **DAUTEL, Hans**
**12163 Berlin (DE)**

(56) Entgegenhaltungen:
**WO-A-02/28186** **WO-A-02/30201**
**WO-A-02/30202** **WO-A-02/43494**
**WO-A-02/087338**

- **DATABASE CROPU [Online] XP002282238 gefunden im STN-INTERNATIONAL Database accession no. 2001-89046 & JP 2001 139403 A (JUKA-LIFETEK) 22. Mai 2001 (2001-05-22)**
- **DATABASE CHEMABS [Online] CHEMICAL ABSTRACTS SERVICE, COLUMBUS, OHIO, US; E.H.ZECK: "Investigations on the green vegetable bug (Nezara viridula Linn.)" XP002282239 gefunden im STN-INTERNATIONAL Database accession no. 28: 17481 CA & AGR.GAZ.N.S.WALES, Bd. 44, Nr. 594, 1992, Seiten 675-682,**
- **N.MENCKEN ET AL.: "Repellent Efficacy of a Combination Containing Imidacloprid and Permethrin against Sand Flies (Phlebotomus papatasi) on Dogs" PARASITOLOGY RESEARCH, Bd. 90, Juli 2003 (2003-07), Seiten S108-S111, XP008031057**
- **DATABASE WPI Section Ch, Week 200348 Derwent Publications Ltd., London, GB; Class C07, AN 2003-508680 XP002282241 & JP 2003 047384 A (YUKO YAKUHIN KOGYO KK) 18. Februar 2003 (2003-02-18)**
- **PATENT ABSTRACTS OF JAPAN Bd. 2003, Nr. 07, 3. Juli 2003 (2003-07-03) & JP 2003 063911 A (NISHIMOTO KOICHI), 5. März 2003 (2003-03-05)**
- **DATABASE CROPU [Online] R.ARTHER ET AL.: "Comparative evaluation of topically applied imidacloprid, permethrin, selamectin and fipronil for control of fleas and ticks on dogs" XP002282240 gefunden im STN-INTERNATIONAL Database accession no. 2002-81364 & PROC.AM.ASSOC.VET.PARASITOL. 46 MEET., 2001, Seite 38,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 587 368 B1

Printed by Jouve, 75001 PARIS (FR)

## Beschreibung

Die vorliegende Erfindung betrifft die Verwendung einer Arthropoden repellierenden Komponente aus der Klasse der Pyrethroide/Pyrethrine in Kombination mit einem Agonisten des nicotinergen Acetylcholinrezeptoren von Arthropoden zur effizienten und lang-anhaltenden Repellierung von Arthropoden, bei warmblütigen Tieren.

Die Verwendung von topischen Formulierungen enthaltend Permethrin, (3-phenoxyphenyl) Methyl 3-(2,2-dichloroethenyl)-2,2-dimethylcyclopropanecarboxylate, (CAS No [52645-53-1] zur Bekämpfung von parasitierenden Insekten an Tieren ist bekannt (vgl. z.B. WO 95/17090, JP-07 247 203, EP-A-567 368, EP-A-461 962, US-5 236 954 und US-5 074 252).

Agonisten der nicotinergen Acetylcholinrezeptoren von Insekten sind bekannt, z.B. aus den Europäischen Offenlegungsschriften Nr. 464 830, 428 941, 425 978, 386 565, 383 091, 375 907, 364 844, 315 826, 25 738, 25 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 25 072; US-Patentschriften Nr. 5 034 524, 4 948 798, 4 918 086, 5 039 686, 5 034 404; PCT-Anmeldungen Nr. WO 91/17 659, 92/4965; Französische Anmeldung Nr. 2611 114; Brasilianische Anmeldung Nr. 88 03 621. Die Verwendung von Spot-on-Formulierungen enthaltend Agonisten oder Antagonisten der nicotinergen Acetylcholinrezeptoren von Insekten zur Bekämpfung von parasitierenden Insekten an Tieren ist ebenso bekannt (siehe beispielsweise WO 98/27 817, EP-A-682 869 und EP 0 976 328).

Im Stand der Technik wurden auch bereits Kombinationen von Permethrin mit Agonisten oder Antagonisten der nicotinergenen Acetylcholinrezeptoren von Insekten zur Bekämpfung von Parasiten beschrieben (vgl. z.B. CN-1 245 637, WO 00/54 591, US-6 080 796, EP-A-981 955, US-6 033 731, JP-07 089 803). Die arthropoden-repellierende Wirkung von Typ-I Pyrethroiden wurde zuerst beschieben in US-4 178 384 (Pyrethroid insect repellent. Ensing, Kenneth J., 1979, US 4178384, betr.: Repellent gegen Schaben), Matthewson et al. (1981, Screening techniques for the evaluation of chemicals with activity as tick repellents. Matthewson, Michael D.; Hughes, Graham; Macpherson, Ian S.; Bernard, Colette P., Pesticide Science, 12(4), 455-62) und Shemanchuk (1981, Repellent action of permethrin, cypermethrin, and resmethrin against black flies (Simulium species) attacking cattle. Shemanchuk, Joseph A., Pesticide Science, 12 (4), 412-16) beschrieben die Repellentwirkung von Typ I und Typ II Pyrethroiden gegen Zecken bzw. gegen Fliegen.

E.H. Zeck beschreibt die abschreckende Wirkung einer Mischung von Pyrethrum-Staub mit Nikotinstaub auf die Grüne Reiswanze (Nezara viridula Linn.) (Agr.Gaz. N.S. Wales, 44, 594, 675-82, (1933).

Der Nachteil der Spot-on-Formulierungen auf z.B. Permethrin-Basis liegt in der geringen Wirksamkeit gegen Flöhe, Mücken und Fliegen.

Spot-on-Formulierungen auf Basis von Agonisten oder Antagonisten der nicotinergen Acetylcholinrezeptoren (siehe z.B. WO 96/17520) weisen in der Regel sehr gute Wirksamkeit gegen Insekten auf. Sie haben jedoch den Nachteil, dass sie gegen Zecken praktisch unwirksam sind und keine repellierende Wirkung zeigen.

Aus diesem Grunde war bislang für eine erfolgreiche Zecken- und Flohbekämpfung sowie die Abwehr von Mücken und Fliegen eine Mehrfachbehandlung der Tiere mit verschiedenen Formulierungen erforderlich. Aus ökologischen und ökonomischen Gründen ist es wünschenswert, diese Formulierungen durch solche zu ersetzen, die gut hautverträglich sowie toxikologisch unbedenklich sind und sich ferner sich bei einem kleinen Applikationsvolumen (z.B. 0,1 ml/1,0 kg [Körpergewicht des zu behandelnden Tieres]) durch ihre gute Langzeitwirkung von mindestens drei bis vier Wochen, vor allem gegen Zecken, Flöhe, Mücken und Fliegen auszeichnen. Weiterhin sollte eine solche Formulierung in allen Klimazonen eine ausreichende Lagerungsstabilität aufweisen, üblicherweise mindestens drei Jahre z.B. in den herkömmlichen Spot-on-Tuben.

WO 02/087338 beschreibt die Bereitstellung einer haut- und umweltverträglichen, anwenderfreundlichen, gegen parasitierende Insekten, insbesondere gegen Zecken und Flöhe, wirksamen Formulierung für die dermale Applikation, enthaltend Permethrin und Agonisten oder Antagonisten der nicotinergen Acetylcholinrezeptoren von Insekten.

Es wurde nun überraschenderweise gefunden, dass Mittel, die Wirkstoffe aus der Gruppe der Pyrethroide/ Pyrethrine in Kombination mit Wirkstoffen, die am Arthropoden-Nicotinrezeptor agonistisch wirken, enthalten, sehr gute repellierende Eigenschaften gegenüber Arthropoden, wie z.B. Zecken, Mücken und Fliegen aufweisen, die über den repellierenden Effekt von Formulierungen, die Pyrethroid/Pyrethrin alleine enthalten, hinausgehen. Dies betrifft die relativen Kontaktzeiten der Ektoparasiten mit dem behandelten Tier. Dabei ist, wie aus vergleichenden in-vitro Untersuchungen hervorgeht, dieser Effekt nicht auf die Formulierung zurückzuführen.

Diese Kombinationsformulierungen des unten näher beschriebenen Typs verhindern überraschenderweise sehr effizient den akuten Befall und damit die potentielle Übertragung von Krankheitserregern durch Arthropoden, insbesondere Zecken, Mücken und saugende Fliegen.

Die vorliegende Erfindung betrifft die

1. Verwendung eines Pyrethroids oder Pyrethrins in Kombination mit einem nicotinischen Agonisten zur Repellierung

von Arthropoden bei warmblütigen Tieren.

2. Verwendung gemäß Punkt 1, wobei das Pyrethroid ausgewählt wird aus der Gruppe:

I. Typ-I Pyrethroide

II. Typ-II Pyrethroide

III. Nicht-Ester-Pyrethroide

IV. Natürlichen Pyrethrine

3. Verwendung gemäß Punkt 1, wobei der nicotinische Agonist ausgewählt wird aus der Gruppe:

V. Neonicotinoide

VI. Nithiazin

VII. Spinosyne

4. Verwendung gemäß Punkt 1 zur Repellierung von Zecken, Flöhen, Mücken und/oder Fliegen an warmblütigen Tieren.

5. Verfahren zur Repellierung von Arthropoden von warmblütigen Tieren, bei dem man ein Pyrethroid oder Pyrethrin in Kombination mit einem nicotinischen Agonisten topisch auf den Warmblüter appliziert.

Die erfindungsgemäßen Mittel sind vorzugsweise flüssig und eignen sich für die dermale Applikation, insbesondere als sogenannte Pour-on- oder Spot-on-Formulierungen. Andere Applikationsformen sind denkbar (s.u.).

Sie enthalten das Pyrethroid oder Pyrethrin üblicherweise in folgenden Mengen:

1. Typ-I Pyrethroide wie z.B. Permethrin: 15-75 Gew.-%, bevorzugt 33 - 55 Gew.-%

II. Typ-II Pyrethroide wie z.B. Cypermethrin: 1-20 Gew.-%, bevorzugt 5-15 Gew.%.

III. Nicht-Ester-Pyrethroide wie z.B. Etofenprox, Silafluofen: 15 - 75 Gew.-%, bevorzugt 40 - 60 Gew.-%.

IV. Natürliche Pyrethrine wie z.B. Pyrethrin I, Jasmolin I, Cinnerin I, Pyrethrin II, Jasmolin II, Cinnerin II: 25 - 75 Gew.-%, bevorzugt 30 - 50 Gew.-%.

Die erfindungsgemäß verwendbaren Mittel enthalten einen Wirkstoff aus der Klasse der nicotinischen Agonisten V-VII in folgenden Mengen:

V. Neonicotinoide mit 1-25 Gew.-%, bevorzugt 5-15 Gew.-%. Beispielhaft seien hier genannt: Imidacloprid, Thiacloprid, Clothianidin, Nitenpyram, Dinotefuran, Thiamethoxam

VI. Nithiazin mit 20 - 40 Gew.-%, bevorzugt 25 - 35 Gew.-%.

VII. Spinosyne mit 1-25 Gew.-%, bevorzugt 5-15 Gew.-%. Beispielhaft seien hier genannt: Spinosad, Butyl-Spinosad.

Weiterhin enthalten die erfindungsgemäß verwendbaren Mittel in der Regel übliche Lösung- und Spreitmittel sowie ggf. übliche Hilfsstoffe.

Die Angaben in Gewichtsprozent beziehen sich auf das Gesamtgewicht.

Die Einteilung der Pyrethroide/Pyrethrine in Typ-I Pyrethroide, Typ-II Pyrethroide, Nicht-Ester-Pyrethroide und natürliche Pyrethrine ist näher erläutert in Encyclopedic Reference of Parasitology 2nd ed., Disease, Treatment, Therapy, (H. Mehlhorn ed.), 2001, Seiten 91-96, worauf ausdrücklich Bezug genommen wird.

Typ-I Pyrethroide sind z.B. Allethrin, Bioallethrin, Permethrin, Phenothrin, Resmethrin, Tetramethrin.

Type-II Pyrethroide sind z.B. alpha-Cypermethrin, Cyfluthrin, Cyhalothrin, Cypermethrin, Deltamethrin, Fenvalerate, Flucythrinate, Flumethrin, tau-Fluvalinate.

Nicht-Ester-Pyrethroide sind z.B. Etofenprox, Silafluofen.

Natürliche Pyrethrine sind z.B. Pyrethrin I, Pyrethrin II, Cinerin I, Cinerin II, Jasmolin I, Jasmolin II

Als Agonisten der nicotinergen Acetylcholinrezeptoren von Insekten werden vorzugsweise die Neonicotinoide genannt.

Unter Neonicotinoiden sollen insbesondere Verbindungen der Formel (I) verstanden werden:

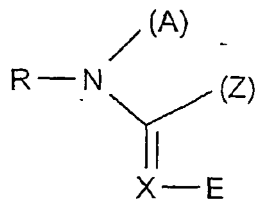

(I),

in welcher

R für Wasserstoff, gegebenenfalls substituierte Reste der Gruppe Acyl, Alkyl, Aryl, Aralkyl, Heteroaryl, Heteroarylalkyl oder Heterocyclylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

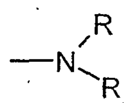

steht,
wobei
R für gleiche oder verschiedene Reste steht und die oben angegebene Bedeutung hat,oder Z für eine bifunktionelle Gruppe steht, die mit dem Rest A oder dem Rest X verknüpft ist.

Besonders bevorzugt sind Verbindungen der Formel (I), in welcher die Reste folgende Bedeutung haben:

R steht für Wasserstoff sowie für gegebenenfalls substituierte Reste aus der Reihe Acyl, Alkyl, Aryl, Aralkyl, Heteroaryl, Heteroarylalkyl, Heterocyclylalkyl.
Als Acylreste seien genannt Formyl, ($C_{1-8}$-Alkyl)-carbonyl, ($C_{6-10}$-Aryl)-carbonyl, ($C_{1-8}$-Alkyl)-sulfonyl, ($C_{6-10}$-Aryl)-sulfonyl, ($C_{1-8}$-Alkyl)-($C_{6-10}$-Aryl)-phosphoryl, die ihrerseits substituiert sein können.
Als Alkyl seien genannt $C_{1-10}$-Alkyl, insbesondere $C_{1-4}$-Alkyl, im einzelnen Methyl, Ethyl, 1-Propyl, sec.- oder t.-Butyl, die ihrerseits substituiert sein können.
Aryl ist insbesondere $C_{6-10}$-Aryl, als Beispiele seien genannt Phenyl, Naphthyl, insbesondere Phenyl.
Aralkyl ist insbesondere ($C_{6-10}$-Aryl)-($C_{1-4}$-Alkyl), als Beispiele seien genannt Phenylmethyl, Phenethyl.
Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.
Heteroarylalkyl ist insbesondere Heteroaryl-($C_{1-4}$-Alkyl), wobei Heteroaryl wie vorstehend definiert ist. Als Beispiele seien genannt Heteroarylmethyl, Heteroarylethyl mit bis zu 6 Ringatomen und N, O, S, insbesondere N als Heteroatomen.
Heterocyclyl ist insbesondere ein ungesättigter aber nicht aromatischer oder gesättigter Heterocyclus mit bis zu 6 Ringatomen, enthaltend bis zu 3 Heteroatome ausgewählt aus N, O, S, zum Beispiel Tetrahydrofuryl.
Heterocyclylalkyl ist insbesondere Heterocyclyl-$C_{1-2}$-Alkyl, z. B.: Tetrahydrofuranylmethyl und Tetrahydrofuranylethyl.

Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano;

Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und 1-propylamin und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo ($-SO_3H$) Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamin wie Chlorpyridylamino und Chlorpyridylmethylamino.

A steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Hexahydro-1,3,5-triazin, Morpholin und Oxadiazin genannt, die gegebenenfalls bevorzugt durch Methyl substituiert sein können.

E steht für einen elektronentziehenden Rest, wobei insbesondere $NO_2$ CN, Halogenalkylcarbonyl wie Halogen-$C_{1-4}$-alkylcarbonyl mit 1 bis 9 Halogenatomen, insbesondere $COCF_3$, sowie $C_{1-4}$-Alkylsulfonyl und Halogen-$C_{1-4}$-alkylsulfonyl mit 1 bis 9 Halogenatomen, insbesondere $SO_2CF_3$ genannt seien.

X steht für -CH= oder -N=

Z steht für gegebenenfalls substituierte Reste Alkyl, -OR, -SR, -NRR, wobei R und die Substituenten bevorzugt die oben angegebene Bedeutung haben.

Z kann außer dem obengenannten Ring gemeinsam mit dem Atom, an welches es gebunden ist und dem Rest

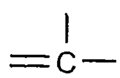

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II), (III) und (IV) genannt:

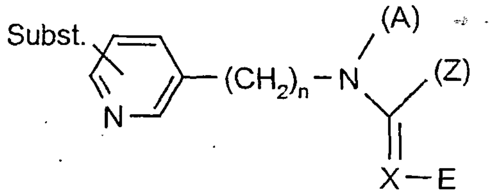

(II),

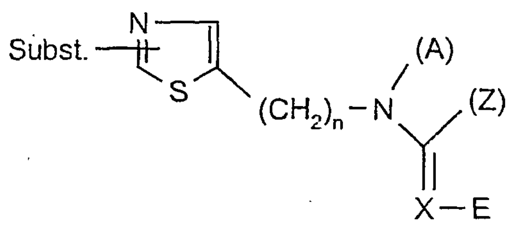

(III),

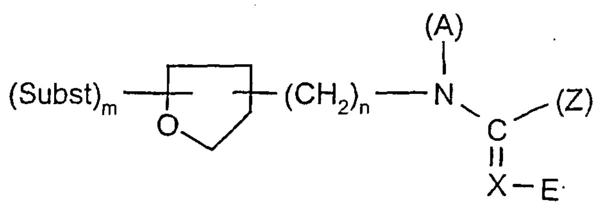

(IV)

in welchen

n für 1 oder 2 steht,

m für 0, 1 oder 2 steht,

Subst. für einen der oben aufgeführten Substituenten, insbesonders für Halogen, ganz besonders für Chlor, steht,

A, Z, X und E die oben angegebenen Bedeutungen haben.

Im einzelnen seien folgende Verbindungen genannt:

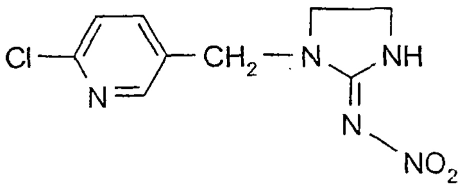

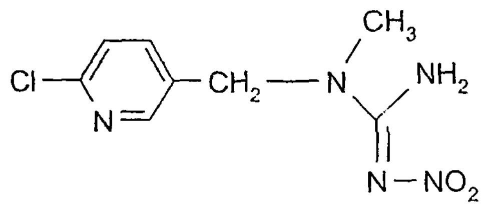

Imidacloprid

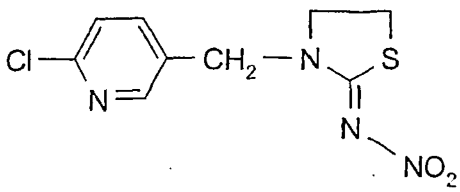

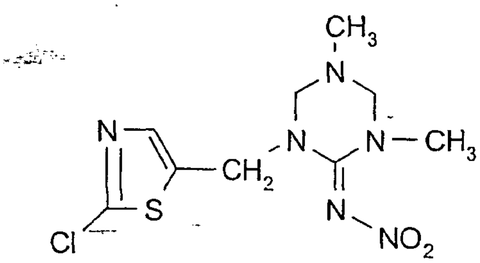

AKD 1022

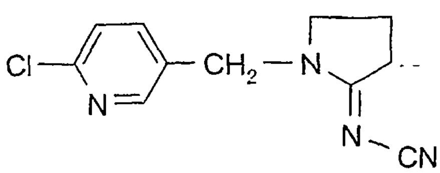

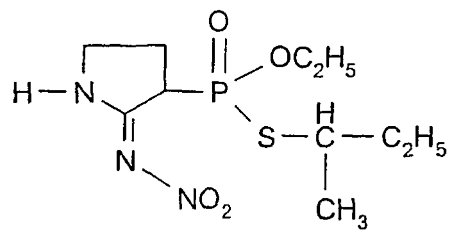

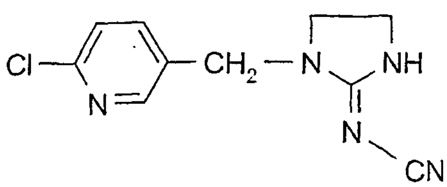

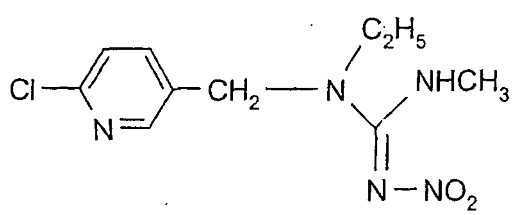

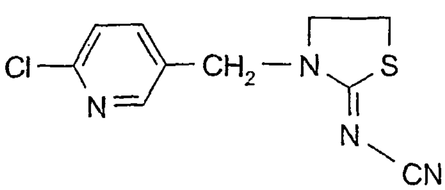

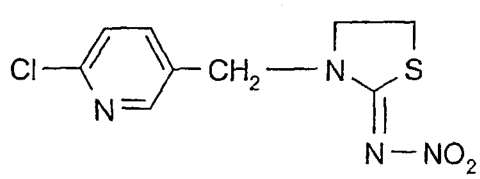

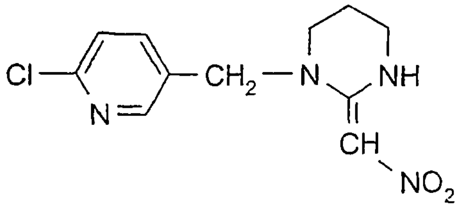

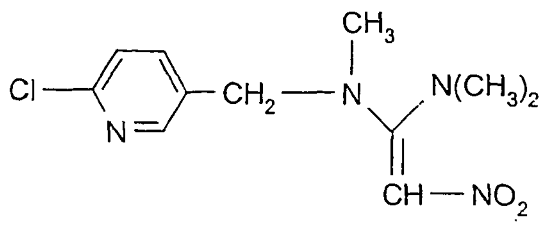

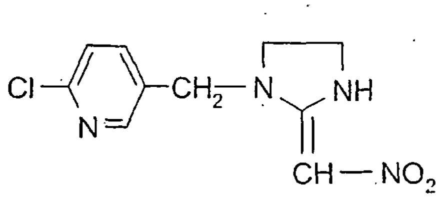

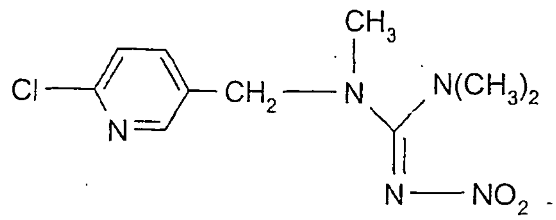

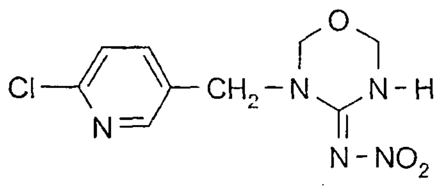

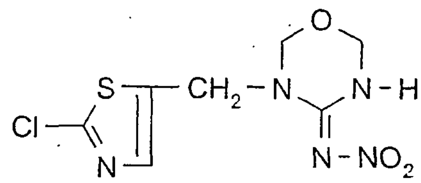

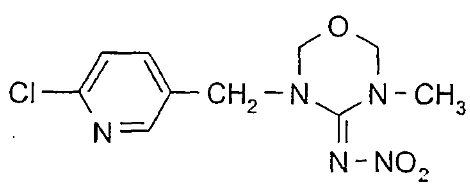

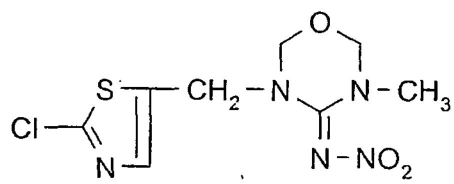

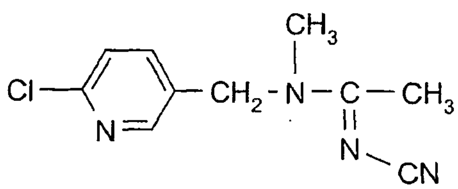

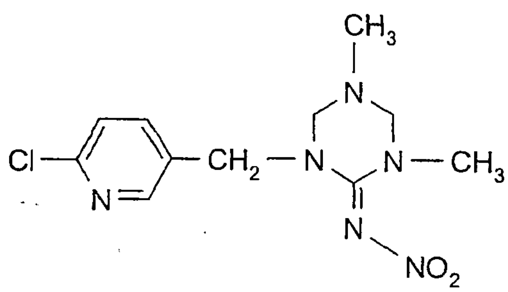

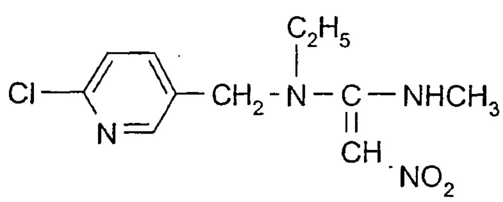

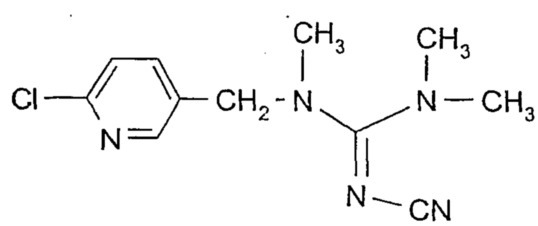

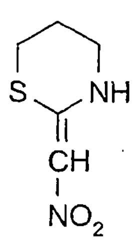

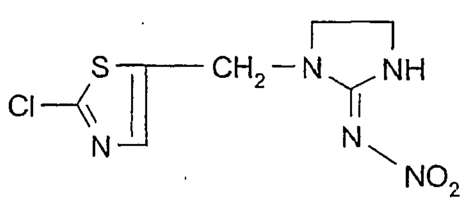

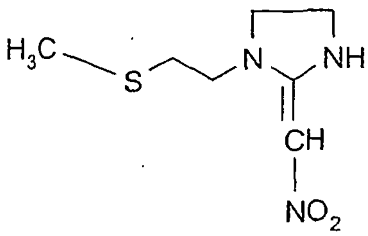

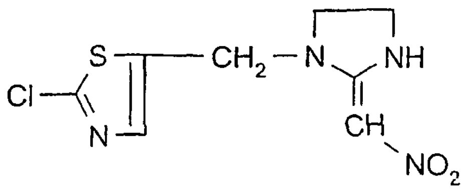

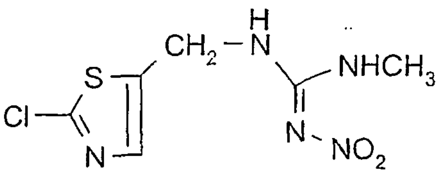

Ti435

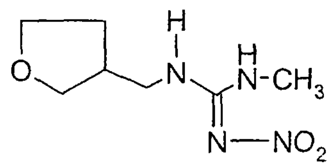

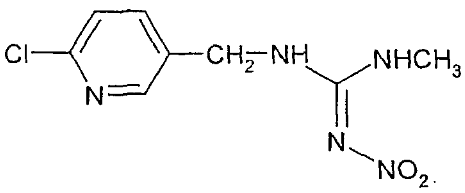

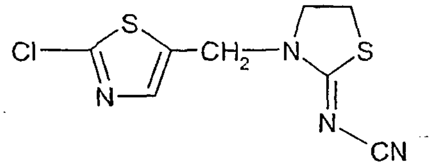

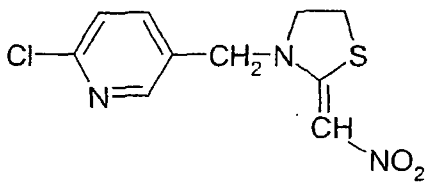

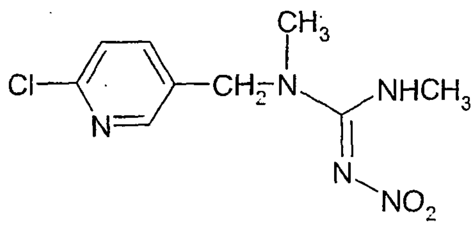

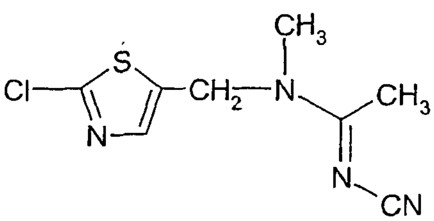

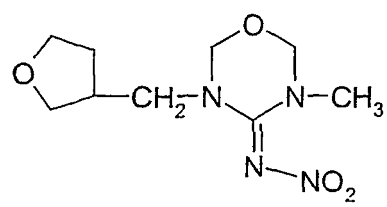

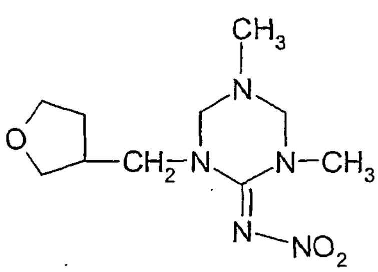

Im einzelnen seien folgende besonders bevorzugte Verbindungen genannt:

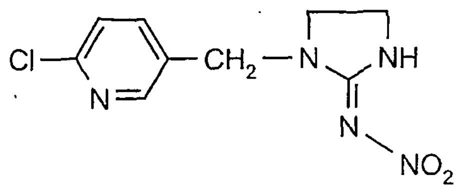

Imidacloprid

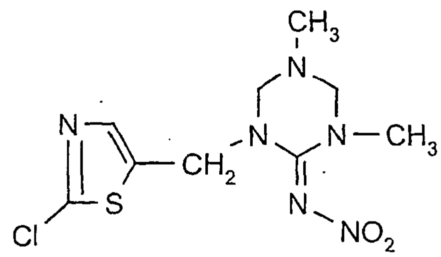

AKD 1022

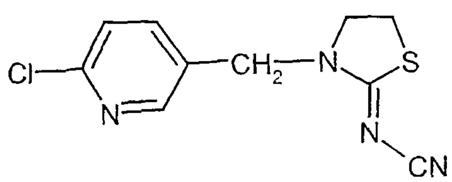

Thiacloprid

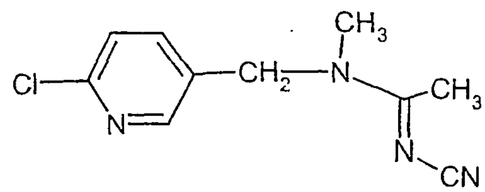

Acetamiprid

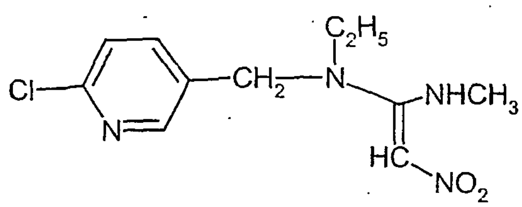

Nitenpyram

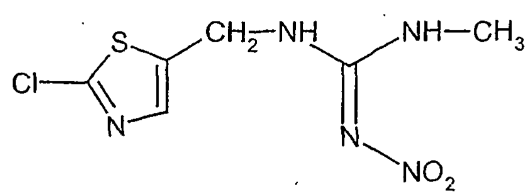

Clothianidin

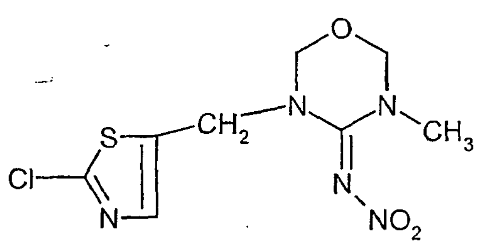

Thiamethoxam (Diacloden)

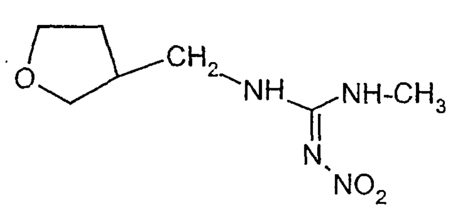

Dinotefuran

Neben nicotinischen Agonisten aus der Gruppe der Neonicotinoide können erfindungsgemäß auch andere nicotinische Agonisten eingesetzt werden.

Beispielhaft seien hier genannt Verbindungen aus der Gruppe der Spinosyne, insbesondere Spinosyn A und D

Spinosyn A

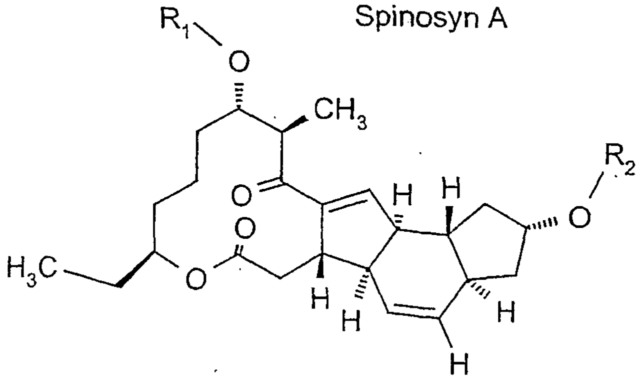

$R_1$ =

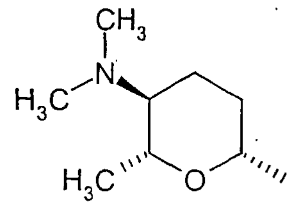

Spinosyn D

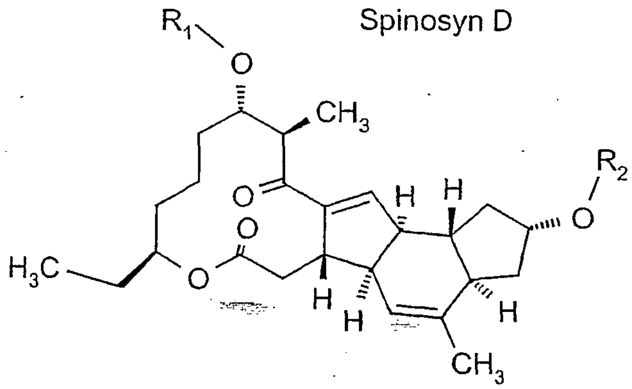

$R_2$ =

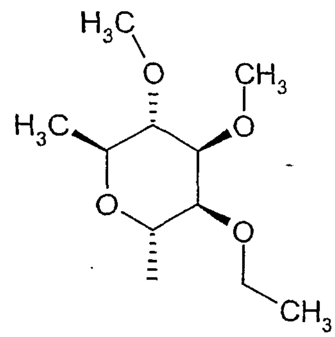

wie beschrieben in Boeck et al. in EP 375316 A1 and Deamicis et al. in WO 97/00265 A1, auf die genannten Dokumente wird ausdrücklich Bezug genommen.

Ebenfalls als Spinosyne werden hier verstanden synthetische und semi-synthetische Derivate der natürlichen Spinosyne bzw. Derivate die aus gentechnisch modifizierten Stämmen von z.B. Saccharopolyspora Spezies gewonnen werden, wie beschrieben in WO 02/77004 und WO 02/77005, auf die genannten Dokumente wird ausdrücklich Bezug genommen.

Beispielhaft genannt seien Verbindungen der Formeln I und II wobei $R^3$ ein Glykosid ($R^3 = R^1$) ist, $R^4$ ist H, OH oder Alkoxy (üblicherweise mit 1 bis 8, bevorzugt 1 bis 4 Kohlenstoffatomen); $R^5$ ist H, Methyl, $R^6$ und $R^7$ sind H oder zur Doppelbindung oder zu einer Epoxygruppe kombiniert, $R^8$ in Formel I ist trans-1-butenyl, 1,3-butadienyl, Butyl, 3-hydroxy-butenyl, Propyl, 1-propenyl, 1,2-epoxy-1-butyl, 3-oxo-1-butenyl, $CH_3CH(OCH_3)CH{=}CH-$, $CH_3CH{=}CHCH(CH_2CO_2CH_3)-$, oder $CH_3CH{=}CHCH[CH_2CON(CH_3)_2]-$; $R^9$ ist H oder Glykosid ($R^9{=}R^2$).

I

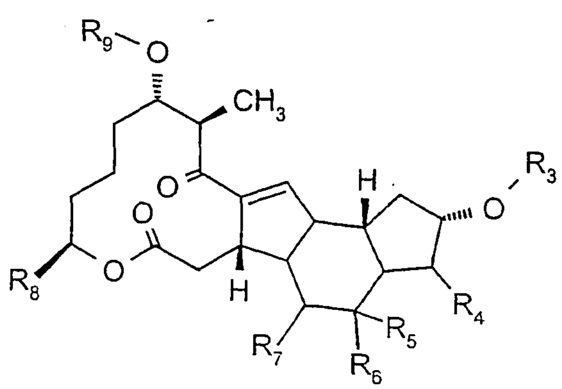

$R_1$ =

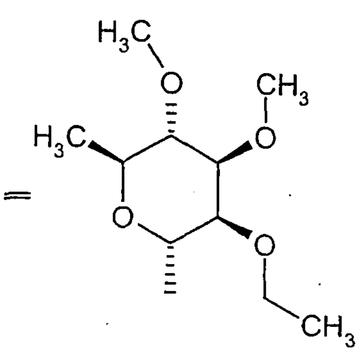

II

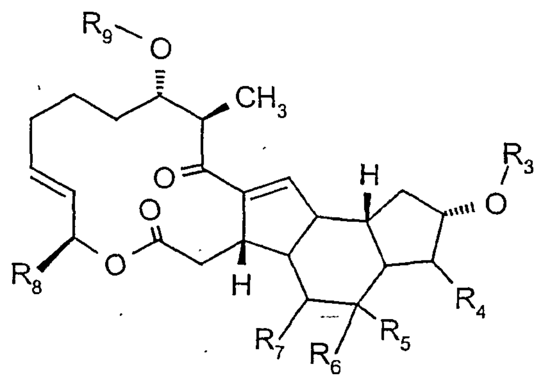

$R_2$ =

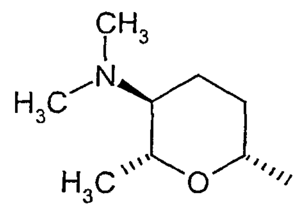

Andere am nicotinischen Rezeptor als Agonisten wirksame Verbindungen; die ebenfalls erfolgreich mit Verbindungen der Gruppe 1 kombiniert werden können, sind beispielsweise Nicotin oder Nithiazin

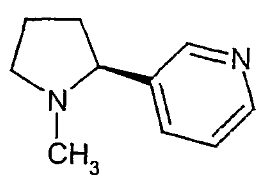

Nicotin

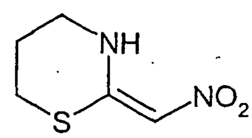

Nithiazin

Der repellierende Effekt der erfindungsgemäß verwendeten Kombination von Wirkstoffen aus der Gruppe der nicotinischen Agonisten in Kombination mit Wirkstoffen der Gruppe der Pyrethroide/Pyrethrine ist überraschenderweise besser als von den Wirkungen der Einzelkomponenten zu erwarten war. Durch Anwendung dieser Mittel können daher die Aufwandmengen an Wirkstoff reduziert sowie die Langzeitwirkung erhöht werden. Ihre Anwendung bringt demzufolge ökonomische und ökologische Vorteile.

Die erfindungsgemäß verwendeten Kombinationen eignen sich hervorragend für den Einsatz bei der Parasitenabwehr und zur Verhinderung der Übertragung von Krankheitserregern, die von solchen Parasiten übertragen werden. Die Parasiten können direkt am Tier abgewehrt werden.

Als Parasiten seien genannt:

Aus der Ordnung der Anoplura z.B. Haematopinus spp., Linognathus spp., Solenopotes spp., Pediculus spp., Pthirus spp.;

aus der Ordnung der Mallophaga z.B. Trimenopon spp., Menopon spp., Eomenacanthus spp., Menacanthus spp., Trichodectes spp., Felicola spp., Damalinea spp., Bovicola spp;

aus der Ordnung der Diptera z.B. Aedes spp., Culex spp., Simulium spp., Phlebotomus spp., Lutzomyia spp., Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp., Pulex spp.

Aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;
aus der Ordnung der Mesostigmata z.B. Dermanyssus spp., Ornithonyssus spp., Pneumonyssus spp..

Aus der Ordnung der Prostigmata z.B. Cheyletiella spp., Psorergates spp., Myobia spp., Demodex spp., Neotrombicula spp.;

aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Cytodites spp., Laminosioptes spp..

Erfindungsgemäß werden die Mittel zur Repellierung von Arthropoden, vorzugsweise von Zecken, Flöhen, Mücken und Fliegen bei warmblütigen Tieren eingesetzt.

Tiere sind z.B. Zucht oder Nutztiere: Säugetiere wie Rinder, Pferde, Schafe, Schweine, Ziegen, Kamele, Wasserbüffel, Esel, Kaninchen, Damwild, Rentiere, Pelztiere wie Nerze, Chinchilla, Waschbär; Vögel, wie z.B. Hühner, Gänse, Puten, Enten, und Strauße.

Weiterhin sind dies Labor- und Versuchstiere, wie z.B. Mäuse, Ratten, Meerschweinchen, Goldhamster, Hunde und Katzen.

Besonders bevorzugt ist der Einsatz bei Hobbytieren, wie z.B. bei Hunden und Katzen.

Da die behandelten Tiere in der Regel auch eine gewisse Menge des eingesetzten Mittels in der Umgebung verteilen, z.B. durch Reibung oder mit Debris, tritt die Wirkung der erfindungsgemäßen Mittel gegebenenfalls nicht nur direkt am Tier sondern auch in entsprechendem Maße in deren Umgebung ein.

Selbstverständlich können die erfindungsgemäß verwendeten Mittel neben den oben genannten Wirkstoffen noch zusätzliche geeignete Wirkstoffe enthalten.

Als Beispiele seien wachstumsheinmende Wirkstoffe und Synergisten genannt, z.B. Pyriproxyfen {2-[1-methyl-2-(4-phenoxyphenoxy)-ethoxy]-pyridine CAS Nr.: 95737-68-1}, Methopren [(E,E)-1-methylethyl 11-methoxy-3,7,11-trimethyl-2,4-dodecadienoate CAS Nr.: 40596-69-8] und Triflumuron {2-chloro-N-[[[4-(trifluoromethoxy)phenyl]amino]carbonyl]benzamide CAS Nr.: 64628-44-0}.

Denkbar ist auch der Zusatz von weiteren repellent wirkenden Stoffen, wie DEET (Diethyltoluamid), Bayrepel® (CAS-Name: 1-Piperidinecarboxylic acid, 2-(2-hydroxyethyl)-, 1-methylpropylester), 2-(Octylthio)-ethanol oder 3-(N-Acetyl-N-butylamino)propionsäureethyl.

Die Anwendung am Tier erfolgt direkt oder in Form von geeigneten Zubereitungen in aller Regel dermal.

Da der Repellentmechanismus der Pyrethroide/Pyrethrine eine Kontaktmöglichkeit mit dem Wirkstoff erfordert, ist es empfehlenswert die Wirkstoffe auf der ganzen zu schützenden Fläche zu verteilen, beispielsweise auf allen Körperteilen der behandelten Tiere. Für die Repellentwirkung ist eine Hautpenetration der Wirkstoffe dabei eher nachteilig, da die in die Haut eingedrungenen Wirkstoffe nicht mehr für die Repellentwirkung zur Verfügung stehen.

Die dermale Anwendung geschieht z.B. in Form des Sprühens (Sprayen) oder Aufgießens (pour-on and spot-on).

Geeignete Zubereitungen sind:

Lösungen oder Konzentrate zur Verabreichung nach Verdünnung zum Gebrauch auf der Haut oder in Körperhöhlen, Aufgußformulierungen, Gele;

Emulsionen und Suspensionen, halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Aerosole und wirkstoffhaltige Formkörper.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methylpyrrolidon, 2-Pyrrolidon sowie Gemische derselben.

Die Wirkstoffe lassen sich gegebenenfalls auch in physiologisch verträglichen pflanzlichen oder synthetischen Ölen, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, Polyvinylalkohol, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Lösungen können direkt angewendet werden. Konzentrate werden nach vorheriger Verdünnung auf die Anwendungskonzentration angewendet.

Lösungen können auf die Haut aufgeträufelt, aufgestrichen, eingerieben, aufgespritzt oder aufgesprüht werden.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdik-

kungsmittel versetzt werden, dass eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere . Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, cyclische Carbonate wie Propylencarbonat, Ethylencarbonat, aromatische und/oder aliphatische Kohlenwasserstoffe, pflanzliche oder synthetische Öle, DMF, Dimethylacetamid, n-Alkylpyrrolidone wie n-Methylpyrrolidon, n-Butyl- oder n-Octylpyrrolidon, N-Methylpyrrolidon, 2-Pyrrolidon, 2,2-Dimethyl-4-oxy-methylen-1,3-dioxolan und Glycerinformal.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, bzw. deren Copolymerisate mit Polyethern, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylenglykolpelargonat, Ester einer verzweigten Fettsäure mittlerer Kettenlänge mit gesättigten Fettalkoholen der Kettenlänge $C_{16-18}$, Isopropylmyristat, Isopropylpalmitat, Capryl/Caprinsäureester von gesättigten Fettalkoholen der Kettenlänge $C_{12-18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Titanoxid, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffe, die gegen pathogene Endoparasiten wirken, vorliegen.

Besonders geeignet, insbesondere für Permethrin-haltige Mittel, sind die in WO 02/087338 beschriebenen Formulierungen.

Diese enthalten: N-Methylpyrrolidon in einem Anteil von 27,5 bis 62,5 Gew.-%, bevorzugt 35 bis 50 Gew.-%, besonders bevorzugt 40 bis 45 Gew.-%.

Antioxidantien in einem Anteil von 0 - 0,5 Gew.-%, bevorzugt 0,05 - 0,25 Gew.-%, besonders bevorzugt 0,05 -0,15 Gew.-%. Es kommen alle üblichen Antioxidantien in Frage, bevorzugt sind phenolische Antioxidantien wie z.B. Butylhydröxytoluol, Butylhydroxyanisol, Tocopherol.

Organische Säure in einem Anteil von 0 - 0,5 Gew.-%, bevorzugt 0,05 - 0,25 Gew.-%, besonders bevorzugt 0,05 -0,15 Gew.-%. Zum Einsatz eignen sich alle pharmazeutisch verträglichen organischen Säuren, insbesondere Carbonsäuren, wie z.B. Citronensäure, Weinsäure, Milchsäure, Bernsteinsäure und Apfelsäure. Besonders bevorzugt sind die organischen Säuren Citronensäure und Apfelsäure. Ganz besonders bevorzugt ist Citronensäure. Ihre Menge kann insbesondere im Bereich 0,05 bis 0,25 breit variiert werden. Wobei die Mengen im Bereich 0,075 - 0,15 % wiederum besonders bevorzugt werden.

Co-Lösungsmittel-Mengen in einem Anteil von 2,5-10 Gew.-%, bevorzugt 2,5 - 7,5 Gew.-%, besonders bevorzugt 3,5 - 6,0 Gew.-%.

Als Co-Lösungsmittel kommen organische Lösungsmittel mit einem Siedepunkt >80°C und einem Flammenpunkt >75°C in Betracht. Bevorzugt haben die Co-Lösungsmitteln eine spreitende Wirkung. In diesem Zusammenhang sei auf höhersiedende aliphatische sowie aromatische Alkohole, aliphatische Polyether, aliphatische und/oder aromatische Ester, cyclische und/oder acyclische Carbonate hingewiesen.

Co-Lösungsmittel sind vorzugsweise aliphatische acyclische oder cyclische Ether bzw. Polyether sowie Fettsäureester insbesondere Triglyceride eingesetzt.

Beispielhaft genannt sind Ether bzw. Polyether beispielsweise aus der Reihe Diethylenglykolmonoethylther, Dipropylenglykolmonomethylether, Tetrahydrofurfurylalkohol und Tetrahydrofurfurylethoxylat, wobei die beiden letztgenannten besonders zu bevorzugen sind; Fettsäureester sowie Triglyceride, beispielsweise Isopropylmyristat, Miglyol 810, Miglyol 812, Miglyol 818, Miglyol 829, Miglyol 840 und Miglyol 8810 (zur Definition der Miglyole siehe beispielsweise H.P. Fiedler Lexikon der Hilfsstoffe für Pharmazie, Kosmetik und angrenzende Gebiete, Seiten 1008-1009, Bd. 2, Edito Cantor Verlag Aulendorf (1996)).

Die mit den genannten Co-Lösungsmitteln modifizierten Mittel zeichnen sich durch ihre sehr gute Haut- und Augenverträglichkeit, ausgezeichnete biologische Wirksamkeit sowie durch ihr günstiges Kältestabilitätsverhalten in den üblichen Single-dose-Applikationstuben aus.

Neben den oben aufgeführten Bestandteilen können die erfindungsgemäße Mittel weitere übliche, pharmazeutisch annehmbare Hilfsstoffe enthalten. Als solche seien beispielsweise genannt: Spreitmittel und Tenside.

Spreitmittel sind beispielsweise spreitende Öle wie Adipinsäure-di-2-ethylhexylester, Isopropylmyristat, Dipropylenglykolpelargonat, cyclische und acyclische Silikonöle, wie Dimetikone und ferner deren Co- und Terpolymerisate mit Ethylenoxid, Propylenoxid und Formalin, Fettsäureester, Triglyceride, Fettalkohole.

Als Tenside seien genannt: Nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Die erfindungsgemäß verwendeten Mittel können nach üblichen Verfahren hergestellt werden, beispielsweise

in dem man die Wirkstoffe unter Rühren mit den weiteren Bestandteilen vermischt und eine Lösung herstellt. Diese kann gegebenenfalls filtriert werden. Zur Abfüllung eignen sich beispielsweise Kunststofftuben.

Die bevorzugten Applikationsvolumina für die in WO 02/087338 beschriebenen Formulierungen liegen bei 0,075-0,25 ml/1,0 kg [Körpergewicht des zu behandelnden Tieres], vorzugsweise 0,1-0,15 ml/1,0 kg [Körpergewicht des zu behandelnden Tieres].

Sie eignen sich hervorragend zum Abfüllen und Ausbieten in lagerungskritischen Behältern, wie z.B. der "Single dose Polypropylenkunststofftuben" der Wandstärke von 300-500 μm und des Abfüllvolumens 1,0 bis 4,0 ml.

Die Mittel sind weiterhin ausgezeichnet hautverträglich, weisen eine geringe Toxizität auf und sind aufgrund ihrer biologischen Abbaubarkeit umweltverträglich.

## Beispiele

### Beispiel 1

Eine homogene Spot-on-Lösung bestehend aus

| | |
|---|---|
| 45 g | Permethrin mit 40 % cis und 60 % trans-Isomerenanteil |
| 10 g | Imidacloprid (1-[(6-Chlor-3-pyridinyl)methyl]-N-nitro-2-imidazolidinimin) der Fa. Bayer AG |
| 44,8 g | N-Methylpyrrolidon |
| 0,1 g | Citronensäure |
| 0,1 g | BHT (Butylhydroxytoluol) |

### Beispiel 2

Eine homogene Spot on Lösung bestehend aus

| | |
|---|---|
| 45 g | Permethrin mit 40 % cis und 60 % trans-Isomerenanteil |
| 10 g | Imidacloprid |
| 40,8 g | N-Methylpyrrolidon |
| 4,0 g | Wasser |
| 0,1 g | Citronensäure |
| 0,1 g | BHT |

### Beispiele 3

Eine homogene Spot on Lösung bestehend aus

| | |
|---|---|
| 45 g | Permethrin mit 40 % cis und 60 % trans-Isomerenanteil |
| 10 g | Ti 435, Chlothianidine Fa. Takeda AG |
| 44,8 g | N-Methylpyrrolidon |
| 0,1 g | Citronensäure |
| 0,1 g | BHT |

### Beispiel 4

Eine homogene Spot on Lösung bestehend aus

| | |
|---|---|
| 45 g | Permethrin mit 40 % cis und 60 % trans-Isomerenanteil |
| 10g | Diacloden (Thiamethoxam) der Fa. Syngenta AG |
| 44,8 g | N-Methylpyrrolidon |
| 0,1 g | Citronensäure |
| 0,1 g | BHT |

**Beispiel 5**

Eine homogene Spot on Lösung bestehend aus

| | |
|---|---|
| 45 g | Permethrin mit 40 % cis und 60 % trans-Isomerenanteil |
| 10 g | Spinosad (8.5 g Spinosyn A; 1.5 g Spinosyn D) Fa. Dow Agrosciences |
| 44,8 g | N-Methylpyrrolidon |
| 0,1 g | Citronensäure |
| 0,1 g | BHT |

**Beispiel 6**

Eine homogene Spot on Lösung bestehend aus

| | |
|---|---|
| 45 g | Permethrin mit 40 % cis und 60 % trans-Isomerenanteil |
| 20 g | Nithiazin Fa. Shell AG |
| 34,8 g | N-Methylpyrrolidon |
| 0,1 g | Citronensäure |
| 0,1 g | BHT |

**Beispiel 7**

Eine homogene Spot on Lösung.bestehend aus

| | |
|---|---|
| 45 g | Permethrin mit 40 % cis und 60 % trans-Isomerenanteil |
| 10 g | Ti 435, Chlothianidine Fa. Takeda AG |
| 39,8 g | N-Methylpyrrolidon |
| 0,1 g | Citronensäure |
| 0,1 g | BHT |
| 5,0 g | Tetrahydrofurfurylalkohol |

**Beispiel 8**

Eine homogene Spot on Lösung bestehend aus

| | |
|---|---|
| 45 g | Permethrin mit 40 % cis und 60 % trans-Isomerenanteil |
| 10 g | Diacloden (Thiamethoxam) der Fa. Syngenta AG |
| 39,8 g | N-Methylpyrrolidon |
| 0,1 g | Citronensäure |
| 0,1 g | BHT |
| 5,0 g | Tetrahydrofurfurylethoxylat |

**Beispiel 9**

Eine homogene Spot on Lösung bestehend aus

| | |
|---|---|
| 45 g | Permethrin mit 40 % cis und 60 % trans-Isomerenanteil |
| 10 g | Spinosad (8.5 g Spinosyn A; 1.5 g Spinosyn D) Fa. Dow Agrosciences |
| 39,8 g | N-Methylpyrrolidon |
| 0,1 g | Citronensäure |
| 0,1g | BHT |
| 5,0 g | Tetrahydrofurfurylethoxylat |

**Beispiel 10**

Eine homogene Spot on Lösung bestehend aus

| | |
|---|---|
| 45 g | Permethrin mit 40 % cis und 60 % trans-Isomerenanteil |
| 20 g | Nithiazin Fa. Shell AG |
| 29,8 g | N-Methylpyrrolidon |
| 0,1 g | Citronensäure |
| 0,1 g | BHT |
| 5,0 g | Tetrahydrofurfurylethoxylat |

**Beispiel 11**

Eine homogene Spot-on-Lösung bestehend aus

| | |
|---|---|
| 10 g | α-Cypermethrin |
| 10 g | Imidacloprid |
| 79,8 g | N-Methylpyrrolidon |
| 0,1 g | Citronensäure |
| 0,1 g | BHT (Butylhydroxytoluol) |

**Beispiel 12**

Eine homogene Spot on Lösung bestehend aus

| | |
|---|---|
| 10g | α-Cypermethrin |
| 10 g | Imidacloprid |
| 75,8 g | N-Methylpyrrolidon |
| 4,0 g | Wasser |
| 0,1 g | Citronensäure |
| 0,1 g | BHT |

**Beispiel 13**

Eine homogene Spot on Lösung bestehend aus

| | |
|---|---|
| 10 g | α-Cypermethrin |
| 10 g | Ti 435, Chlothianidine Fa. Takeda AG |
| 79,8 g | N-Methylpyrrolidon |
| 0,1 g | Citronensäure |
| 0,1 g | BHT |

**Beispiel 14**

Eine homogene Spot on Lösung bestehend aus

| | |
|---|---|
| 10 g | α-Cypermethrin |
| 10 g | Diacloden (Thiamethoxam) der Fa. Syngenta AG |
| 79,8 g | N-Methylpyrrolidon |
| 0,1 g | Citronensäure |
| 0,1 g | BHT |

**Beispiel 15**

Eine homogene Spot on Lösung bestehend aus

| | |
|---|---|
| 10 g | α-Cypermethrin |
| 10 g | Spinosad (8.5 g Spinosyn A; 1.5 g Spinosyn D) Fa. Dow Agrosciences |
| 79,8 g | N-Methylpyrrolidon |
| 0,1 g | Citronensäure |
| 0,1 g | BHT |

**Beispiel 16**

Eine homogene Spot on Lösung bestehend aus

| | |
|---|---|
| 10 g | α-Cypermethrin |
| 20 g | Nithiazin Fa. Shell AG |
| 69,8 g | N-Methylpyrrolidon |
| 0,1 g | Citronensäure |
| 0,1 g | BHT |

**Beispiel 17**

Eine homogene Spot on Lösung bestehend aus

| | |
|---|---|
| 10 g | α-Cypermethrin |
| 10 g | Ti 435, Chlothianidine Fa. Takeda AG |
| 74,8 g | N-Methylpyrrolidon |
| 0,1 g | Citronensäure |
| 0,1 g | BHT |
| 5,0 g | Tetrahydrofurfurylalkohol |

**Beispiel 18**

Eine homogene Spot on Lösung bestehend aus

| | |
|---|---|
| 10 g | α-Cypermethrin |
| 10 g | Diacloden (Thiamethoxam) der Fa. Syngenta AG |
| 74,8 g | N-Methylpyrrolidon |
| 0,1 g | Citronensäure |
| 0,1 g | BHT |
| 5,0 g | Tetrahydrofurfurylethoxylat |

**Beispiel 19**

Eine homogene Spot on Lösung bestehend aus

| | |
|---|---|
| 10 g | α-Cypermethrin |
| 10 g | Spinosad (8.5 g Spinosyn A; 1.5 g Spinosyn D) Fa. Dow Agrosciences |
| 74,8 g | N-Methylpyrrolidon |
| 0,1 g | Citronensäure |
| 0,1 g | BHT |
| 5,0 g | Tetrahydrofurfurylethoxylat |

**Beispiel 20**

Eine homogene Spot on Lösung bestehend aus

| | |
|---|---|
| 10 g | α-Cypermethrin |
| 20 g | Nithiazin Fa. Shell AG |
| 64,8 g | N-Methylpyrrolidon |
| 0,1 g | Citronensäure |
| 0,1 g | BHT |
| 5,0 g | Tetrahydrofurfurylethoxylat |

**Beispiel 21**

Eine homogene Spot-on-Lösung bestehend aus

| | |
|---|---|
| 45 g | Etofenprox |
| 10 g | Imidacloprid |
| 44,8 g | N-Methylpyrrolidon |
| 0,1 g | Citronensäure |
| 0,1 g | BHT (Butylhydroxytoluol) |

**Beispiel 22**

Eine homogene Spot on Lösung bestehend aus

| | |
|---|---|
| 45 g | Etofenprox |
| 10 g | Imidacloprid |
| 40,8 g | N-Methylpyrrolidon |
| 4,0 g | Wasser |
| 0,1 g | Citronensäure |
| 0,1 g | BHT |

**Beispiel 23**

Eine homogene Spot on Lösung bestehend aus

| | |
|---|---|
| 45 g | Etofenprox |
| 10 g | Ti 435, Chlothianidine Fa. Takeda AG |
| 44,8 g | N-Methylpyrrolidon |
| 0,1 g | Citronensäure |
| 0,1 g | BHT |

**Beispiel 24**

Eine homogene Spot on Lösung bestehend aus

| | |
|---|---|
| 45 g | Etofenprox |
| 10 g | Diacloden (Thiamethoxam) der Fa. Syngenta AG |
| 44,8 g | N-Methylpyrrolidon |
| 0,1 g | Citronensäure |
| 0,1 g | BHT |

**Beispiel 25**

Eine homogene Spot on Lösung bestehend aus

| | |
|---|---|
| 45 g | Etofenprox |
| 10 g | Spinosad (8.5 g Spinosyn A; 1.5 g Spinosyn D) Fa. Dow Agrosciences |
| 44,8 g | N-Methylpyrrolidon |
| 0,1 g | Citronensäure |
| 0,1 g | BHT |

**Beispiel 26**

Eine homogene Spot on Lösung bestehend aus

| | |
|---|---|
| 45 g | Etofenprox |
| 20 g | Nithiazin Fa. Shell AG |
| 34,8 g | N-Methylpyrrolidon |
| 0,1 g | Citronensäure |
| 0,1 g | BHT |

**Beispiel 27**

Eine homogene Spot on Lösung bestehend aus

| | |
|---|---|
| 45 g | Etofenprox |
| 10 g | Ti 435, Chlothianidine Fa. Takeda AG |
| 39,8 g | N-Methylpyrrolidon |
| 0,1 g | Citronensäure |
| 0,1 g | BHT |
| 5,0 g | Tetrahydrofurfurylalkohol |

**Beispiel 28**

Eine homogene Spot on Lösung bestehend aus

| | |
|---|---|
| 45 g | Etofenprox |
| 10 g | Diacloden (Thiamethoxam) der Fa. Syngenta AG |
| 39,8 g | N-Methylpyrrolidon |
| 0,1 g | Citronensäure |
| 0,1 g | BHT |
| 5,0 g | Tetrahydrofurfurylethoxylat |

**Beispiel 29**

Eine homogene Spot on Lösung bestehend aus

| | |
|---|---|
| 45 g | Etofenprox |
| 10 g | Spinosad (8.5 g Spinosyn A; 1.5 g Spinosyn D) Fa. Dow Agrosciences |
| 39,8 g | N-Methylpyrrolidon |
| 0,1 g | Citronensäure |
| 0,1 g | BHT |
| 5,0 g | Tetrahydrofurfurylethoxylat |

**Beispiel 30**

Eine homogene Spot on Lösung bestehend aus

| | |
|---|---|
| 45 g | Etofenprox |
| 20 g | Nithiazin Fa. Shell AG |
| 29,8 g | N-Methylpyrrolidon |
| 0,1 g | Citronensäure |
| 0,1 g | BHT |
| 5,0 g | Tetrahydrofurfurylethoxylat |

**Beispiel 31**

Eine homogene Spot-on-Lösung bestehend aus

| | |
|---|---|
| 45 g | Pyrethrum Extrakt |
| 10 g | Imidacloprid |
| 44,8 g | N-Methylpyrrolidon |
| 0,1 g | Citronensäure |
| 0,1 g | BHT (Butylhydroxytoluol) |

**Beispiel 32**

Eine homogene Spot on Lösung bestehend aus

| | |
|---|---|
| 45 g | Pyrethrum Extrakt |
| 10 g | Imidacloprid |
| 40,8 g | N-Methylpyrrolidon |
| 4,0 g | Wasser |
| 0,1 g | Citronensäure |
| 0,1 g | BHT |

**Beispiel 33**

Eine homogene Spot on Lösung bestehend aus

| | |
|---|---|
| 45 g | Pyrethrum Extrakt |
| 10 g | Ti 435, Chlothianidine Fa. Takeda AG |
| 44,8 g | N-Methylpyrrolidon |
| 0,1 g | Citronensäure |
| 0,1 g | BHT |

**Beispiel 34**

Eine homogene Spot on Lösung bestehend aus

| | |
|---|---|
| 45 g | Pyrethrum Extrakt |
| 10 g | Diacloden (Thiamethoxam) der Fa. Syngenta AG |
| 44,8 g | N-Methylpyrrolidon |
| 0,1 g | Citronensäure |
| 0,1 g | BHT |

**Beispiel 35**

Eine homogene Spot on Lösung bestehend aus

| | |
|---|---|
| 45 g | Pyrethrum Extrakt |
| 10 g | Spinosad (8.5 g Spinosyn A; 1.5 g Spinosyn D) Fa. Dow Agrosciences |
| 44,8 g | N-Methylpyrrolidon |
| 0,1 g | Citronensäure |
| 0,1 g | BHT |

**Beispiel 36**

Eine homogene Spot on Lösung bestehend aus

| | |
|---|---|
| 45 g | Pyrethrum Extrakt |
| 20 g | Nithiazin Fa. Shell AG |
| 34,8 g | N-Methylpyrrolidon |
| 0,1 g | Citronensäure |
| 0,1 g | BHT |

**Beispiel 37**

Eine homogene Spot on Lösung bestehend aus

| | |
|---|---|
| 45 g | Pyrethrum Extrakt |
| 10 g | Ti 435, Chlothianidine Fa. Takeda AG |
| 39,8 g | N-Methylpyrrolidon |
| 0,1 g | Citronensäure |
| 0,1 g | BHT |
| 5,0 g | Tetrahydrofurfurylalkohol |

**Beispiel 38**

Eine homogene Spot on Lösung bestehend aus

| | |
|---|---|
| 45 g | Pyrethrum Extrakt |
| 10 g | Diacloden (Thiamethoxam) der Fa. Syngenta AG |
| 39,8 g | N-Methylpyrrolidon |
| 0,1 g | Citronensäure |
| 0,1 g | BHT |
| 5,0 g | Tetrahydrofurfurylethoxylat |

**Beispiel 39**

Eine homogene Spot on Lösung bestehend aus

| | |
|---|---|
| 45 g | Pyrethrum Extrakt |
| 10 g | Spinosad (8.5 g Spinosyn A; 1.5 g Spinosyn D) Fa. Dow Agrosciences |
| 39,8 g | N-Methylpyrrolidon |
| 0,1 g | Citronensäure |
| 0,1 g | BHT |
| 5,0 g | Tetrahydrofurfurylethoxylat |

**Beispiel 40**

Eine homogene Spot on Lösung bestehend aus

| | |
|---|---|
| 45 g | Pyrethrum Extrakt |
| 20 g | Nithiazin Fa. Shell AG |
| 29,8 g | N-Methylpyrrolidon |
| 0,1 g | Citronensäure |
| 0,1 g | BHT |
| 5,0 g | Tetrahydrofurfurylethoxylat |

**A. Repellierung von Zecken im Moving-Object Biotest. Vergleich mit Stand der Technik**

Methode

Moving-Object-Bioassay nach Dautel et al. (1999)

Kurzbeschreibung: Einzelne Zecken nähern sich einer erwärmten langsam rotierenden vertikalen Trommel auf einem horizontal stehenden Glasstab. Die Zecken werden von der Wärme der Trommel angelockt und wechseln auf eine Anheftungsstelle auf der rotierenden Trommel über. Wenn auf diese Anheftungsstelle ein Repellent aufgetragen wird, kann der Repellenteffekt entweder i) an einer abnehmenden Zahl von auf die Trommel zustrebenden Zecken, oder ii) durch eine reduzierte Zahl von Zecken, die auf die Anheftungsstelle wechseln oder iii) durch eine steigende Zahl von Zecken, die sich vorzeitig von der Anheftungsstelle wieder fallen lassen, gemessen. Als Vergleich dient eine Trommel mit einer unbehandelten Kontrolle. Es können sowohl Kontakt- als auch Distanz-Repellenzien gemessen werden.

Testbedingungen:

Jeder Testwert wird mit 30 Zecken durchgeführt. Alle Zecken werden einzeln nacheinander in der gleichen Apparatur geprüft. Zu jeder Testreihe wird Kontrolltest mit reinem Lösungsmittel ohne Repellent durchgeführt, um die Grundaktivität der Zecken zu prüfen. Als Grenzaktivität für die Durchführung eines Tests wird das Wechseln von mindestens 70% der Zecken auf die Trommel gewertet. Für jedes Testprodukt wird eine eigene Testirommel verwendet. Nach jeder Testreihe werden alle verwendeten Geräte sorgfältig gereinigt.

Für die Prüfung von Ixodes ricinus und Rhipicephalus sanguineus Zecken wurden folgende Bedingungen eingestellt.

I. ricinus:

Eine Standardtrommel und Anheftungsfläche wurde verwendet (Dautel et al. (1999)). Die Anheftungsfläche lag 1-3 mm oberhalb der Trommeloberfläche. Der Abstand zwischen dem Glasstab von 2 mm Durchmesser und der Anheftungsfläche betrug zwischen 1 und maximal 1,5 mm.

Die Rotationszeit der Trommel betrug zwischen 3,9 und 4,1 s / Umdrehung entsprechend 7,66 - 8,05 cm/s relativ zur Zecke. Die Oberflächentemperatur an der Anheftungsfläche betrug zwischen 34,6 und 35,5˚C. Raumtemperatur und Umgebungsfeuchte lagen zwischen 19,1 - 22,3 ˚C bzw. 43,4 - 78,1 % r.h.

R. sanguineus:

Adulte R. sanguineus haben eine höhere Laufgeschwindigkeit als I. ricinus Nymphen. Daher muss die Anheftungsfläche auf der Trommel derart vergrößert werden, dass eine Kontaktzeit von mindestens 10 sec auch für schnell laufende Exemplare garantiert werden kann. Es dient hier somit die gesamte Trommel als Anheftungsstelle. Aufgrund der geringen Anheftung der Zecken auf Filterpapier wurde die Trommel mit Moltontuch bespannt. Der Abstand zwischen Molton und Glasstab (4 mm Durchmesser) betrug 1-3 mm womit die Zecken zu jeder Zeit in der Lage waren, vom Glasstab auf die Trommel überzuwechseln.

Die Rotationszeit der Trommel betrug zwischen 5,6 und 6,0 s / Umdrehung entsprechend 5,23 - 5,61 cm/s relativ zur Zecke. Die Oberflächentemperatur an der Anheftungsfläche betrug zwischen 35 und 36˚C. Raumtemperatur und Umgebungsfeuchte lagen zwischen 19,4 - 23,5 ˚C bzw. 59,1 - 79,5 % r.h.

Testsubstanzapplikation

Aceton wurde als Lösungsmittel und für die Verdünnungen bei allen Versuchen verwendet. Die Applikation erfolgte 1-2 Stunden vor Versuchsbeginn, um ausreichend Zeit zum Verdunsten des Lösungsmittels zu lassen.
Die Wirkstoffe wurden auf die Filterpapiere mit einer Wegwerfpipette aufgetragen. Eine gleichmäßige Verteilung auf der größeren Oberfläche des Moltontuches wurde mit Hilfe einer Sprühapparatur unter Stickstoffdruck erzielt. Das genaue applizierte Volumen wurde hier durch Rückwiegen ermittelt.

MO-Bioassay

Es wurden nur solche Zecken im Test eingesetzt, die aktiv in einem Glasröhrchen zum oberen Rand kletterten und zügig auf einen Dachshaarpinsel (0 oder 1) wechselten der zum Übertragen der Zecken verwendet wurde. Diese Zecken wurden mit dem Kopf zur Trommel auf den Glasstab gesetzt in einem Abstand von 1,5 cm (I. ricinus) oder 2.5-4 cm (R. sanguineus) zur Spitze des Glasstabs. Die Versuchszeit startete sobald eine Zecke die 1 cm (I. ricinus) bzw. die 2 cm (R. sanguineus) Markierung auf dem Glasstab überquert hatte. Zecken, die vom Pinsel fielen oder sich vor der Markierung vom Glasstab fallen ließen wurden nicht gewertet.
Folgende Zeitabschnitte wurden mittels Stoppuhr festgehalten:

- Zeit vom Überqueren der Marke bis zum Erreichen des Endes des Glasstabes
- Zeit vom Erreichen der Spitze des Glasstabes bis zum Übertritt auf die Trommel
- Zeit, die die Zecke auf dem Filter oder auf dem Moltontuch bleibt bis sie sich fallen lässt oder die behandelte Fläche verlässt.

Für jeden dieser Zeitabschnitte waren maximal 120 sec. vorgesehen. Nach 2 Minuten wurde die Zecke entfernt und der Zeitraum als 120 sec. gewertet.
Eine Gesamtrepellentwirkung relativ zur Kontrolle wurde berechnet, indem alle Zecken zusammengerechnet wurden, die nicht Richtung Trommel gingen, die nicht auf die Trommel wechselten und die sich von der Anheftungsfläche fallen ließen. Alle diese Zecken wurden als repelliert gewertet. Die Repellentwirkung wird wie folgt berechnet:

$$R = 100 - pt/pc * 100,$$

wobei R die Repellentwirkung, pt der Prozentsatz der nicht repellierten Zecken und pc der Prozentsatz der nicht repellierten Kontrollzecken ist.

**Ergebnisse aus Repellentversuchen mit Rhipicephalus sanguineus Zecken - Vergleich mit dem Stand der Technik (Exspot®, Fa. Schering-Plough)**

**Tabelle 1a:** *R. sanguineus:* Aufenthaltszeit [s] auf der behandelten Trommeloberfläche.

| Formulierung (Dosierung) | n | Mittel | SD | 95 % Conf. |
|---|---|---|---|---|
| Kontrolle | 25 | 83,8 | 39,7 | 67,4 - 100,2 |
| Beispiel 1 (17/83 $\mu g/cm^2$) *) | 18 | 5,5 | 3,9 | 3,6 - 7,5 |
| Exspot® (83,3 $\mu g/cm^2$) | 20 | 21,9 | 30,3 | 7,7 - 36,0 |
| *) Der erste Wert bezieht sich auf die Imidacloprid-Menge, der zweite auf die Permethrin-Menge | | | | |

Die Formulierung aus Beispiel 1 zeigt überraschenderweise eine deutlich stärkere Repellentwirkung als der Standard (Exspot® enthält Permethrin als einzigen Wirkstoff). Zecken die auf die Trommel übergehen fallen bei Beispiel 1 signifikant schneller wieder ab als beim Standard. Die Repellentwirkung des Standards wird im vorliegenden Beispiel im Mittel um einen Faktor 4 verstärkt.

**Tabelle 1b:** ***R. sanguineus:*** **Zeitdauer [s] zum Übertritt von der Glasstabspitze auf die Trommel.**

| Formulierung (Dosierung) | n | Mittel | SD | 95 % Conf. |
|---|---|---|---|---|
| Kontrolle | 30 | 3,5 | 6,2 | 1,2 - 5,8 |
| Beispiel 1 (17/83 μg/$cm^2$) *) | 30 | 7,9 | 22,1 | 0,3 - 16,2 |
| Exspot® (83,3 μg/$cm^2$) | 27 | 2,8 | 6,9 | 0,0 - 5,5 |
| *) Der erste Wert bezieht sich auf die Imidacloprid-Menge, der zweite auf die Permethrin-Menge | | | | |

Ein weiteres Zeichen für die verbesserte Repellentwirkung ist das verzögerte Übertreten vom Glasstab auf die Trommel. Auch hier zeigen Zecken bei Beispiel 1 gegenüber dem Standard ein im Mittel um den Faktor 3 verlängertes Zeitintervall. Der Standard liegt hierbei im Bereich einer Kontrolle.

**Tabelle 2:** ***R. sanguineus:*** **MO-Biotest-Bewertung: Erfindungsgemäße Formulierung und Stand der Technik (Exspot®, Fa. Schering-Plough) gegen Kontrolle**

| Dosis | Kontrolle | Beispiel 1 | *Kontrolle* | *Exspot®* |
|---|---|---|---|---|
| | | 16,6/83,1 μg/$cm^2$ | | *8,3μglcm²* |
| Nicht repellierte Zecken | 27 | 0 | 25 | 3 |
| Repellierte Zecken | 3 | 30 | 5 | 27 |
| % nicht repelliert | 90,0 | 0,0 | 83,3 | 10 |
| Repellentwirkung [% der Kontrolle] | | 100,0 | | *88,0* |

Gegenüber der jeweiligen Kontrolle zeigt die erfindungsgemäße Formulierung aus Beispiel 1 eine 100 %ige Repellentwirkung im relevanten Dosisbereich für eine topikal gleichmäßig verteilte Formulierung nach spot-on Anwendung. Das bekannte Handelsprodukt ist unter den gleichen Bedingungen überraschenderweise nicht in der Lage alle Zecken zu repellieren.

**Ergebnisse aus Repellentversuchen mit Ixodes ricinus Zecken - Vergleich mit dem Stand der Technik (Exspot®, Fa. Schering-Plough)**

Tabelle 3: *I. ricinus:* **MO-Biotest-Bewertung: Erfindungsgemäße Formulierung und Stand der Technik (Exspot®, Fa. Schering-Plough) gegen Kontrollen**

| Formulierung/Dosierung | | Beispiel 1 (erfindungsgemäß) | | | | |
|---|---|---|---|---|---|---|
| Verhaltensparameter | Kontrolle | 1,9/9,3 µg /cm² | *5/25 µg/cm²* | 19/93 µg/cm² | 190/930 µg/cm² | Kontrolle |
| Nicht repellierte Zecken | 28 | 20,5 | *17* | 18 | 15 | 27 |
| Repellierte Zecken | 2 | 9,5 | *13* | 12 | 15 | 3 |
| % nicht repelliert | 93,3 | 6,3 | *56,7* | 60,0 | 50,0 | 90,0 |
| Repellentwirkung [%] | | 25,3 | *39,3* | 33,3 | 44,4 | |

| Formulierung/Dosierung | | Exspot® | | | | |
|---|---|---|---|---|---|---|
| Verhaltensparameter | Kontrolle | 9,3 µg/cm² | *25 µg/cm²* | 93 µg/cm² | 930 µg/cm² | Kontrolle |
| Nicht repellierte Zecken | 26 | 24 | *22* | 18 | 15 | *28* |
| Repellierte Zecken | 4 | 6 | *8* | 12 | 15 | *2* |
| % nicht repelliert | 86,7 | 80,0 | *73,3* | 60,0 | 50,0 | *93,3* |
| Repellentwirkung [%] | | 7,7 | *21,4* | 30,8 | 42,3 | |

Auch bei Ixodes, einer Zecke, die durch Exspot® nicht gut repelliert wird, zeigt das erfindungsgemäße Beispiel überraschenderweise eine deutlich verbesserte Repellentwirkung. Insbesondere bei geringen Dosierungen, wie sie zu Beginn einer Behandlung an von der Auftragsstelle des Spot-ons weiter entfernten Körperoberflächen sowie am Ende der Wirkdauer auf dem gesamten Tier zu erwarten sind zeigt das erfindungsgemäße Beispiel Repellenz im gleichen Bereich wie in den höheren Dosierungen, während die Kurve der Repellentwirkung beim Stand der Technik bereits um das 6-fache abgenommen hat.

Erfindungsgemäße Formulierungen zeigen bei gleicher Aufwandmenge somit in den wichtigen Parametern Übertrittswahrscheinlichkeit auf die Oberfläche, Aufenthaltsdauer auf der Oberfläche sowie bei der Wirksamkeit bei geringeren Dosierungen eine deutlich verbesserte Repellentwirkung gegen Zecken gegenüber dem Stand der Technik.

**B. Mortalität von Zecken im Moving-Object Biotest. (Vergleichsbeispiel, nicht unter den Umfang der Erfindung fallend)**

Methode

Bestimmung der Endmortalität nach Kurzzeitkontakt im Moving-Object-Biotest

Kurzbeschreibung: Nach Exposition wurden die Zecken einzeln in Eppendorfgefäße mit gelochtem Deckel überführt und bei 90 % r.h. und 20°C aufbewahrt. Nach 24 Stunden und nach 7 Tagen wurden die Zecken mittels Binokular untersucht. Zecken, die zur koordinierten Bewegung in der Lage waren, wurden als lebendig gewertet. Zecken, die nur kleine Bewegungen mit den Tarsen oder Mundwerkzeugen machten oder unfähig zum Laufen waren, wurden als moribund gewertet. Zecken, die nach CO2-Stimulus oder Starklichtimpuls unbeweglich blieben, wurden als tot gewertet.

Die Untersuchungen sollten zeigen, ob es einen Zusammenhang zwischen Expositionszeit (=Aufenthaltszeit auf der behandelten Trommelfläche während eines MO-Biotests) und der Mortalität bei unterschiedlichen Konzentrationen verschiedener Formulierungen im Vergleich zum Stand der Technik gibt.

**Tabelle 1:** ***R. sanguineus:*** **Mortalität (d 7) und Kontaktzeiten bei verschiedenen Konzentrationen von Test Formulierungen im MO-Biotest.**

| ***Formulierung*** | ***Dosierung*** (a.i.) | ***Mortalität*** n | % | Mittel | ***Kontaktzeit [s]*** SD | 95% Conf. |
|---|---|---|---|---|---|---|
| Beispiel 1 | 16,6/83,1 μg/cm$^2$ | 21 | 70 % | 4,2 | 4,4 | 2,2-6,2 |
| Exspot® | 83,3 μg/cm$^2$ | 16 | 53 % | 24,7 | 33,5 | 6,9-42,6 |

**Tabelle 2:** ***I. ricinus:*** **Mortalität (d 7)und Kontaktzeiten bei verschiedenen Konzentrationen von Test Formulierungen im MO-Biotest.**

| **Formulierung** | **Dosierung** (a.i.) | **Mortalität** n | % | **kontaktzeit [s]** Mittel | SD | 95% Conf. |
|---|---|---|---|---|---|---|
| Beispiel 1 | 19/93 μg/cm$^2$ *) | 27 | 90 | 33,7 | 30,6 | 21,6-45,9 |
| Exspot® | 93 μg/cm$^2$ | 26 | 87 | 41,9 | 31,1 | 29,3-54,4 |
| *) Der erste Wert bezieht sich auf die Imidacloprid-Menge, der zweite auf die Permethrin-Menge | | | | | | |

Sowohl Ixodes als auch Rhipicephalus Zecken werden zu einem höheren Prozentsatz abgetötet, nachdem sie Kontakt mit der Trommeloberfläche hatten. Die für diese höhere Mortalität benötigte mittlere Kontaktzeit war dabei bei erfindungsgemäßen Formulierungen sogar noch kürzer als beim Stand der Technik.

Somit schützen die erfmdungsgemäßen Formulierungen zusätzlich dadurch, dass repellierte Zecken auch nach kurzen Kontaktzeiten von deutlich weniger als 1 Minuten bereits abgetötet werden und somit weitere Wirte nicht mehr von repellierten Zecken befallen werden können.

**Patentansprüche**

**1.** Verwendung eines Pyrethroids oder Pyrethrins in Kombination mit einem Agonisten der nicotinergen Acetylcholinrezeptoren von Arthropoden zur Repellierung von Arthropoden bei warmblütigen Tieren.

**2.** Verwendung gemäß Anspruch 1, wobei das Pyrethroid ausgewählt wird aus der Gruppe:

I. Typ-1 Pyrethroide
II. Typ-II Pyrethroide
III. Nicht-Ester-Pyrethroide

IV. Natürlichen Pyrethrine

**3.** Verwendung gemäß Anspruch 1, wobei der nicotinische Agonist ausgewählt wird aus der Gruppe:

V. Neonicotinoide
VI. Nithiazin
VII. Spinosyne

**4.** Verwendung gemäß Anspruch 1 zur Repellierung von Zecken, Flöhen, Mücken und/oder Fliegen an warmblütigen Tieren.

**5.** Verfahren zur Repellierung von Arthropoden von warmblütigen Tieren, bei dem man ein Pyrethroid oder Pyrethrin in Kombination mit einem nicotinischen Agonisten topisch auf den Warmblüter appliziert.

## Claims

**1.** Use of a pyrethroid or pyrethrin in combination with an agonist of the nicotinergic acetylcholine receptors of arthropods for repelling arthropods in warm-blooded species.

**2.** Use according to Claim 1, wherein the pyrethroid is selected from amongst the following groups:

I. Type I pyrethroids
II. Type II pyrethroids
III. Non-ester pyrethroids
IV. Natural pyrethrins

**3.** Use according to Claim 1, wherein the nicotinic agonist is selected from amongst the following groups:

V. Neonicotinoids
VI. Nithiazine
VII. Spinosyns

**4.** Use according to Claim 1 for repelling ticks, fleas, midges and/or flies on warm-blooded species.

**5.** Method for repelling arthropods from warm-blooded species, in which a pyrethroid or pyrethrin in combination with a nicotinic agonist is applied topically to the warm-blooded animal.

## Revendications

**1.** Utilisation d'un pyréthroïde ou de pyréthrine en combinaison avec un agoniste des récepteurs nicotinergiques de l'acétylcholine d'arthropodes pour produire un effet répulsif sur des arthropodes parasites d'animaux à sang chaud.

**2.** Utilisation suivant la revendication 1, dans laquelle le pyréthroïde est choisi dans le groupe :

I. pyréthroïdes de type I
II. pyréthroïdes de type II
III. pyréthroïdes non-esters
IV. pyréthrines naturelles.

**3.** Utilisation suivant la revendication 1, dans laquelle l'agoniste nicotinique est choisi dans le groupe :

V. néonicotinoïdes
VI. nithiazine
VII. spinosynes

**4.** Utilisation suivant la revendication 1, pour produire un effet répulsif sur des tiques, des puces, des moustiques et/ou

des mouches parasites d'animaux à sang chaud.

5. Procédé pour produire un effet répulsif sur des arthropodes parasites d'animaux à sang chaud, dans lequel on applique localement aux animaux à sang chaud un pyréthroïde ou une pyréthrine en combinaison avec un agoniste nicotinique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9517090 A
- JP 7247203 B
- EP 567368 A
- EP 461962 A
- US 5236954 A
- US 5074252 A
- EP 464830 A
- EP 428941 A
- EP 425978 A
- EP 386565 A
- EP 383091 A
- EP 375907 A
- EP 364844 A
- EP 315826 A
- EP 25738 A
- EP 25859 A
- EP 235725 A
- EP 212600 A
- EP 192060 A
- EP 163855 A
- EP 154178 A
- EP 136636 A
- EP 303570 A
- EP 302833 A
- EP 306696 A
- EP 189972 A
- EP 455000 A
- EP 135956 A
- EP 471372 A
- EP 302389 A
- DE 3639877
- DE 3712307
- JP 03220176 A
- JP 02207083 A
- JP 63307857 B
- JP 63287764 B
- JP 03246283 B
- JP 4009371 A
- JP 03279359 B
- JP 0325072 A
- US 5034524 A
- US 4948798 A
- US 4918086 A
- US 5039686 A
- US 5034404 A
- WO 9117659 A
- WO 924965 A
- FR 2611114
- BR 03621
- WO 9827817 A
- EP 682869 A
- EP 0976328 A
- CN 1245637
- WO 0054591 A
- US 6080796 A
- EP 981955 A
- US 6033731 A
- JP 07089803 A
- US 4178384 A
- WO 9617520 A
- WO 02087338 A
- EP 375316 A1, Boeck
- WO 9700265 A1, Deamicis
- WO 0277004 A
- WO 0277005 A


### In der Beschreibung aufgeführte Nicht-Patentliteratur


- **Matthewson, Michael D. ; Hughes, Graham ; Macpherson, Ian S. ; Bernard, Colette P.** *Pesticide Science,* vol. 12 (4), 455-62
- **Shemanchuk, Joseph A.** *Pesticide Science,* vol. 12 (4), 412-16
- *Agr.Gaz. N.S. Wales,* 1933, vol. 44 (594), 675-82
- Disease, Treatment, Therapy. Encyclopedic Reference of Parasitology. 2001, 91-96
- **H.P. Fiedler.** Lexikon der Hilfsstoffe für Pharmazie, Kosmetik und angrenzende Gebiete. Edito Cantor Verlag Aulendorf, 1996, vol. 2, 1008-1009